# EUROPEAN PATENT APPLICATION

(11) **EP 4 310 478 A1**
(43) Date of publication of application: **24.01.2024**
(21) Application number: 22770945.8
(22) Date of filing: 08.02.2022
(51) Int. Cl.: G01N 15/14, C12M 1/00, C12N 5/10, C12Q 1/02, G01N 1/04

(54) **CLOSED-SYSTEM AUTOMATIC SAMPLE SORTING SYSTEM**

(30) Priority: 16.03.2021 JP 2021042749
(71) Applicant: Sony Group Corporation, Tokyo 108-0075 (JP)
(72) Inventor: HAYASHI Yoshihito, Tokyo 108-0075 (JP)
(74) Representative: D Young & Co LLP
(86) International application number: PCT/JP2022/004858
(87) International publication number: WO 2022/196186

(57) **Abstract**

Disclosed in a sample sorting system including: a container in which a sample being sorted is stored; a sorting part that sorts a target biological sample from the sample being sorted; and a storage part used to store the target biological sample in, the container, the sorting part, and the storage part being airtightly connected through a flow channel, and further including a first liquid container that contains a liquid that is caused to flow through the flow channel. This sample sorting system is useful as an automated sample sorting system that enables a plurality of processes of CSC pretreatment to be performed in a sterile closed circuit.

## Description

### TECHNICAL FIELD

The present technology relates to a closed-system automated sample sorting system and a sample sorting device.

### BACKGROUND ART

Gene-modified T cell therapy (CART therapy) has already been put into practical use, and for example, in the United States, Kymriah from Novartis AG was launched in August 2017, Yescarta from Kite Pharma, Inc. (subsidiary of Gilead Sciences, Inc.) was launched in October 2017, and Kymriah was also approved in Japan in March 2019. Cell therapy such as CART therapy and technologies related to cell therapy are fields that are expected to grow further in the future. At present, a method using antibody modified magnetic beads is considered to be the mainstream of the process of recovering immune system cells required for cell therapy. Such a process using magnetic beads allows a large amount of blood sample to be processed at a time, but has problems that fine cell subsets cannot be selectively separated by a single process, it becomes contaminated with a large amount of non-target cells, which results in poor purity, and it is difficult to determine the number of target cells. In other words, in the field of cell therapy such as cancer immunotherapy, it is important to selectively separate and use cells effective for therapy in order to improve the therapy outcome and safety, reduce side effects, and increase the number of adaptable patients, and as a method to achieve the above under a sterile environment, the development of a closed-system cell sorter (CSC) used for cell therapy is in progress (Patent Document 1).

### CITATION LIST

### PATENT DOCUMENT

Patent Document 1: Japanese Patent Application Laid-Open No. 2017-181278

### SUMMARY OF THE INVENTION

### PROBLEMS TO BE SOLVED BY THE INVENTION

Pretreatment (removal of red blood cells and platelets, staining with a fluorescently labeled antibody, cleaning, concentration adjustment, and the like) before a sample taken is put into the CSC, however, has problems that, for example, the pretreatment requires, at present, a great deal of labor and cost, and further requires a large amount of handwork, which increases a risk of contamination. A device capable of performing each process of such pretreatment in a closed system is available, but such a device has no capability of performing all the processes without interruption, and requires work such as manually replacing and reconnecting bags, and handling a sample or a reagent in a safety cabinet. Furthermore, there is no device capable of automatically adjusting concentration at the final stage.

It is therefore an object of the present technology to provide an automated sample sorting system that enables a plurality of processes of CSC pretreatment to be performed in a sterile closed circuit.

### SOLUTIONS TO PROBLEMS

The present technology provides a sample sorting system including: a container in which a sample being sorted is stored; a sorting part that sorts a target biological sample from the sample being sorted; and a storage part used to store the target biological sample in, the container, the sorting part, and the storage part being airtightly connected through a flow channel, and further including a first liquid container that contains a liquid that is caused to flow through the flow channel. In the sample sorting system, the sorting part includes a first filter module that separates a mixture of solids smaller in size than target bioparticles and a mixture containing the target bioparticles, a second filter module that separates a mixture of solids larger in size than the target bioparticles and the mixture containing the target bioparticles, a first reagent container that contains a reagent used to label the target bioparticles or particles other than the target bioparticles, and a chamber used to temporarily hold a sample, a particle, a reagent, a liquid, or a mixture thereof in the flow channel.

In the sorting part in the sample sorting system, a combination of (a), (b), and (c1), a combination of (a), (b), and (c2), or a combination of (a) and (c2) is performed on the sample being sorted flowing from the container, where:
(a) is removal of the mixture of solids smaller in size than the target bioparticles by the first filter module;
(b) is removal of the mixture of solids larger in size than the target bioparticles by the second filter module;
(c1) is processing of labeling the target bioparticles by reaction between the reagent flowing from the first reagent container and the sample being sorted in the chamber and then sorting the labeled particles; and
(c2) is processing of labeling the particles other than the target bioparticles by reaction between the reagent flowing from the first reagent container and the sample being sorted in the chamber and then removing the labeled particles.

The labeling may be performed using beads, and the reagent used to label particles may be a reagent used to bind the beads to the target bioparticles or the particles other than the target bioparticles.

In a case where the beads are used for labeling, in the sorting part in the sample sorting system, a combination of (a), (b), and (c1), a combination of (a), (b), and (c2), or a combination of (a) and (c2) is performed on the sample being sorted flowing from the container, where:
(a) is removal of the mixture of solids smaller in size than the target bioparticles by the first filter module;
(b) is removal of the mixture of solids larger in size than the target bioparticles by the second filter module;
(c1) is processing of binding the beads to the target bioparticles by reaction between the reagent flowing from the first reagent container and the sample being sorted in the chamber and then causing the sample being sorted to flow through the second filter module to sort the target bioparticles bound to the beads as the mixture of solids larger in size than the target bioparticles; and
(c2) is processing of binding the beads to the particles other than the target bioparticles by reaction between the reagent flowing from the first reagent container and the sample being sorted in the chamber and then causing the sample being sorted to flow through the second filter module to remove the particles bound to the beads as the mixture of solids larger in size than the target bioparticles. Here, in a case where the processing (b) is performed after the processing (c1), processing of removing the beads from the target bioparticles is performed after the processing (c1) and before the processing (b).

In a case where the beads are used for labeling, in the sample sorting system, the sorting part may include the first reagent container that contains the reagent used to bind the beads to the target bioparticles, and another reagent container that contains a reagent used to bind the beads to the particles other than the target bioparticles, and in the sorting part, a combination of (a), (b), (c1), and (c2) or a combination of (a), (c1), and (c2) is performed on the sample being sorted flowing from the container, where:
(a) is removal of the mixture of solids smaller in size than the target bioparticles by the first filter module;
(b) is removal of the mixture of solids larger in size than the target bioparticles by the second filter module;
(c1) is processing of binding the beads to the target bioparticles by reaction between the reagent flowing from the first reagent container and the sample being sorted in the chamber and then causing the sample being sorted to flow through the second filter module to sort the target bioparticles bound to the beads as the mixture of solids larger in size than the target bioparticles; and
(c2) is processing of binding the beads to the particles other than the target bioparticles by reaction between the reagent flowing from the another reagent container and the sample being sorted in the chamber and then causing the sample being sorted to flow through the second filter module to remove the particles bound to the beads as the mixture of solids larger in size than the target bioparticles. Here, in a case where the processing (b) and/or the processing (c2) is performed after the processing (c1), processing of removing the beads from the target bioparticles is performed after the processing (c1) and before the processing (b) and/or the
processing (c2).

In the sample sorting system, the first filter module and the second filter module may each include a microchannel that separates and guides solids in a sample introduced from an inlet to two different outlets in accordance with sizes of the solids.

Furthermore, the sample sorting system may further include a labeling part that labels the sample being sorted with a fluorescent dye, and the labeling part may be airtightly connected to at least one of the container or the sorting part.

Moreover, in the sample sorting system, a concentration of the target bioparticles may be adjusted.

Moreover, in the sample sorting system, the liquid may be introduced from the first liquid container into the container, and the liquid may be mixed with the sample being sorted.

Furthermore, the present technology also provides a sample sorting device including: the above-described sample sorting system including a container in which a sample being sorted is stored; a sorting part that sorts a target biological sample from the sample being sorted; and a storage part used to store the target biological sample in, the container, the sorting part, and the storage part being airtightly connected through a flow channel; a light irradiation unit that irradiates the sample being sorted with excitation light; a light detection unit that detects fluorescence emitted from the sample being sorted; and an arithmetic processing unit that calculates sorting information on the basis of a detection result from the light detection unit.

The sample sorting device may include a cell sorter airtightly connected to the above-described sample sorting system, and the cell sorter preferably includes a closed-system cell sorter, and more preferably includes a closed-system cell sorter used for cell therapy.

Moreover, the sample sorting device may further include a culture unit that adjusts the temperature of the storage part.

Furthermore, the sample sorting device may further include a drug feed management unit that controls feed of a drug into the storage part.

### EFFECTS OF THE INVENTION

According to the present technology, it is possible to provide an automated low-cost device that enables a series of processes of CSC pretreatment to be performed in a sterile closed circuit, and furthermore, simultaneously performing some processes that have been separately performed so far makes a process time shorter. The reduction of the process time is not only related to work efficiency but also leads to a reduction of cell damage, and thus is useful for cell therapeutic agent production. Meanwhile, the effects are not necessarily limited to the effects herein described and may be any of the effects described in the present technology.

### BRIEF DESCRIPTION OF DRAWINGS

Fig. 1 is a schematic diagram depicting a configuration example of a sample sorting system according to the present technology.
Fig. 2 is a flowchart depicting a specific example of sample sorting including negative selection using the sample sorting system according to the present technology.
Fig. 3 is a flowchart depicting a specific example of sample sorting including positive selection using the sample sorting system according to the present technology.

### MODE FOR CARRYING OUT THE INVENTION

Hereinafter, a preferred embodiment for carrying out the present technology will be described below with reference to the drawings. The embodiment to be described below shows one of representative embodiments of the present technology, and the scope of the present technology is not narrowly interpreted by the example.

### <1. Sample Sorting System>

### (1) Description of Sample Sorting System of Present Technology

A sample sorting system of the present technology includes: a container in which a sample being sorted is stored; a sorting part that sorts a target biological sample from the sample being sorted; and a storage part used to store the target biological sample in, the container, the sorting part, and the storage part being airtightly connected through a flow channel, and further including a first liquid container that contains a liquid that is caused to flow through the flow channel. The sorting part includes a first filter module that separates a mixture of solids smaller in size than target bioparticles and a mixture containing the target bioparticles, a second filter module that separates a mixture of solids larger in size than the target bioparticles and the mixture containing the target bioparticles, a first reagent container that contains a reagent used to label the target bioparticles or particles other than the target bioparticles, and a chamber used to temporarily hold a sample, a particle, a reagent, a liquid, or a mixture thereof in the flow channel.

In the sample sorting system of the present technology, the solids smaller in size than the target bioparticles and the solids larger in size than the target bioparticles are removed as much as possible from solids contained in the sample being sorted by sequentially using the first filter module and the second filter module. Moreover, in the sample sorting system of the present technology, either the target bioparticles or the particles other than the target bioparticles are labeled with a labeling reagent, thereby separating the target bioparticles and the particles other than the target bioparticles. It is therefore possible to increase a concentration of the target bioparticles in the sample being sorted, that is, it is possible to concentrate the target bioparticles.

### (2) Configuration Example of Sample Sorting System of Present Technology and Example of Sample Sorting Method Using Sample Sorting System

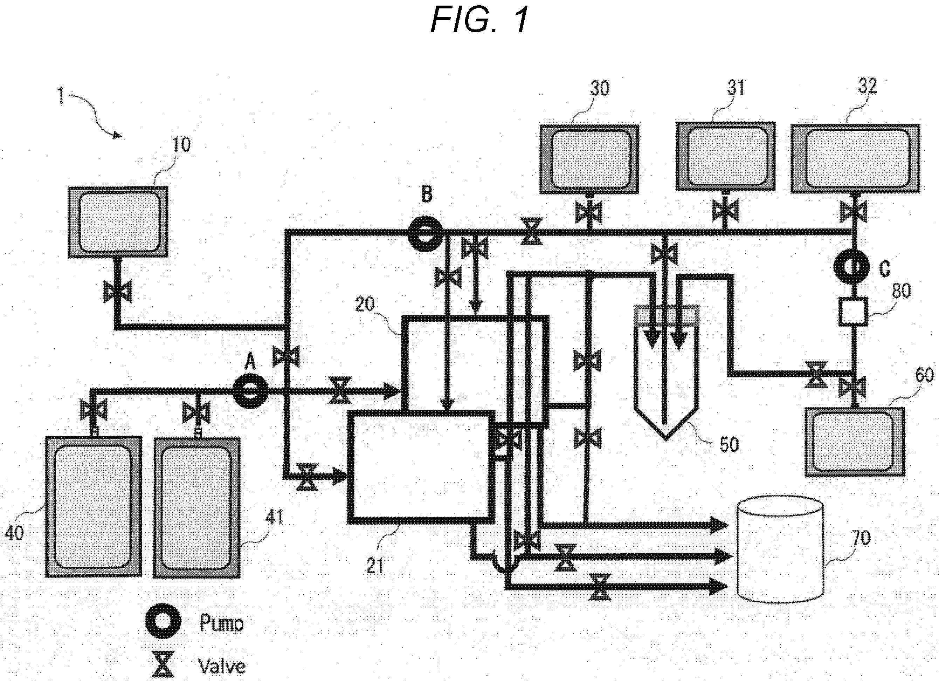

A configuration example of the sample sorting system of the present technology and how to use the sample sorting system will be described below with reference to Fig. 1.

### (i) Configuration Example

The sample sorting system 1 depicted in Fig. 1 includes a container 10 in which the sample being sorted is stored, a first filter module 20, a second filter module 21, a first reagent container 30 that contains the reagent used to label the target bioparticles or the particles other than the target bioparticles, a chamber 50 used to temporarily hold a sample, a particle, a reagent, a liquid or a mixture thereof in a flow channel, a first liquid container 40 that contains a liquid that is caused to flow through the flow channel, and a storage part 60. The sample sorting system of the present technology may include a plurality of liquid containers and a plurality of reagent containers, and the sample sorting system 1 depicted in Fig. 1 includes a second liquid container 41, a second reagent container 31, and a third reagent container 32. The sample sorting system 1 depicted in Fig. 1 further includes a concentration sensor 80 used to sense the concentration of the target bioparticles, and a disposal part 70 used to store waste in.

All the components included in the sample sorting system of the present technology are connected by an airtightly connected flow channel, and a sample, a particle, a reagent, a liquid, or a mixture thereof in the flow channel can be moved by operation of a valve and a pump as depicted in Fig. 1. It is therefore possible to perform all of a plurality of processes for sorting a sample in a sterile closed circuit.

### (ii) Sample being sorted

The sample being sorted is stored in the container 10. The sample being sorted is not particularly limited as long as the sample contains the target bioparticles to be sorted. Since all the plurality of processes for sorting the sample can be performed in a sterile closed circuit, the sample sorting system of the present technology, however, is particularly advantageous in a case where the target bioparticles are cells.

The cells may include animal cells (such as blood cells) and plant cells. The cells may particularly be blood cells or tissue cells. Examples of the blood cells may include, for example, white blood cells (for example, peripheral blood mononuclear cells), red blood cells, and platelets, and the blood cells particularly include the white blood cells. Examples of the white blood cells may include, for example, monocytes (macrophages), lymphocytes, neutrophils, basophils, and eosinophils. The cells may include, for example, floating cells such as T cells and B cells. The tissue cells may include, for example, adherent cells separated from adherent cultured cells or tissues, or the like. Furthermore, the cells may include tumor cells. The cells may be cultured or uncultured. The cells to be the target bioparticles may include, for example, cells for use in therapy or blood cells such as white blood cells. In a case where the target bioparticles are blood cells, the sample being sorted is a blood-derived sample, and examples of the blood-derived sample include a sample obtained by thawing a frozen apheresis sample, a fresh apheresis sample (unfrozen), and a whole blood sample.

In a case where (there is a possibility that) a nucleic acid is contained in the sample being sorted, such as a case where the sample being sorted contains frozen cells, it is desirable to degrade the nucleic acid by mixing a nucleic acid degrading substance with the sample being sorted. Such mixing of the nucleic acid degrading substance can be made by causing, with the nucleic acid degrading substance stored in the second reagent container 31 in advance, the nucleic acid degrading substance to flow from the second reagent container 31 to the container 10. Alternatively, the nucleic acid degrading substance may be caused to flow from the second reagent container 31 throughout the flow channel. It is therefore possible to prevent aggregation of solids in the sample being sorted, and is thus possible to prevent the flow channel and the filter modules from being clogged. As such a nucleic acid degrading substance, a nucleic acid degrading enzyme can be suitably used, and examples of the nucleic acid degrading enzyme include deoxyribonuclease (such as DNase I), ribonuclease, and a mixture thereof. In a case where a solid nucleic acid degrading substance is stored in the second reagent container 31, the solid nucleic acid degrading substance can be used after the liquid is caused to flow from the first liquid container 40 to the second reagent container 31 to dissolve the solid nucleic acid degrading substance.

### (iii) Liquid (Carrier)

The liquid stored in the first liquid container 40 serves as a carrier of the sample being sorted containing the target bioparticles, and is thus a liquid having no harmful effect on the target bioparticles. As such a liquid, it is preferable to use a buffer (buffer solution), and examples of the buffer include normal phosphate-buffered saline (PBS+), phosphate-buffered saline (PBS-) obtained by removing magnesium and calcium from PBS+, physiological saline, a culture medium, a Ringer's solution, and the like. Furthermore, a buffer obtained by adding, to PBS-, magnesium ions at a physiological concentration or by an amount equivalent to or twice the amount of magnesium ions in PBS+ can also be used, and with this composition, it is possible to add divalent ions necessary for enzyme activity while avoiding calcium ions contained in PBS+ from affecting cell viability and the like. Furthermore, albumin, an antibiotic, and glucose at a physiological concentration may be added to such buffers.

### (iv) Liquid (Cleaning Liquid)

In the present technology, as the liquid, a cleaning liquid (a hypochlorous acid solution, a surfactant solution, or the like) for cleaning the components and the flow channel included in the sample sorting system 1 can be used, and this cleaning liquid can be stored in the second liquid container 41, for example. It is possible to cause, as necessary, this cleaning liquid to flow through some or all of the components and the flow channel included in the sample sorting system 1 and then flow into the disposal part.

In particular, this cleaning liquid is suitable for cleaning the container 10 from which the sample being sorted has been removed, and the first filter module 20 and the second filter module 21 through which the sample being sorted has flowed. Furthermore, after the cleaning with the cleaning liquid is finished, in order to prevent damage to the target bioparticles such as cells in the subsequent processes, it is desirable to cause the liquid in the first liquid container 40 to flow to a portion with which the cleaning liquid has come into contact to remove the cleaning liquid.

### (v) Concentrating Target Bioparticle in Sorting Part

A filter module used in the present technology may be any filter module as long as the filter module is capable of separating and guiding a liquid into two outlets in accordance with the sizes of the particles present in the liquid, and a specific structure of the filter module is not particularly limited. For example, examples of the filter module include a hollow fiber filter, and further include a tubular filter further having a tubular structure on the outer side thereof. A liquid component (which may contain small particles) that has flowed out through microholes of the hollow fiber filter or the tubular filter can be guided to another flow channel. A flow channel led out from the outer tube is connected to an outlet through which small-sized particles flow out. On the other hand, the flow in the hollow fiber filter or the tubular filter can be introduced (circulated) into the hollow fiber filter or the tubular filter again via the chamber, and a lead-out channel openable and closable by a valve is provided in the circulation circuit, and the lead-out channel is connected to an outlet through which large-sized particles flow out.

As the filter module used in the present technology, a microchannel can also be used. This microchannel is also referred to as a microchannel device or a microchannel chip. In this microchannel, a sample is guided into a microfluidic channel where the sample flows in parallel with another liquid (such as a buffer solution). Within this channel, precisely arranged island structures can generate fluid lift (force in a direction orthogonal to a main flow) and classify the particles of interest (such as cells) by size. A width of the flow channels in the microchannel chip described above can be adjusted in a range of about 1 to 1000 um. A threshold applied when the solids are separated from the sample by the microchannel can be appropriately determined in accordance with the size of the target bioparticles and the size of the solids to be removed from the sample being sorted.

The first filter module 20 is not particularly limited as long as it is a module capable of separating a mixture of solids smaller in size than the target bioparticles and a mixture containing the target bioparticles. As such a first filter module 20, for example, it is possible to suitably use a microchannel that separates solids in a sample introduced from an inlet into a mixture of solids smaller in size than the target bioparticles and a mixture containing the target bioparticles and guides the resultant mixtures to different outlets.

The second filter module 21 is not particularly limited as long as it is a module capable of separating a mixture of solids larger in size than the target bioparticles and a mixture containing the target bioparticles. As such a second filter module 21, for example, it is possible to suitably use a microchannel that separates solids in a sample introduced from an inlet into a mixture of solids larger in size than the target bioparticles and a mixture containing the target bioparticles and guides the resultant mixtures to different outlets.

In the sample sorting system 1 depicted in Fig. 1, the first filter module 20 and the second filter module 21 are each provided with two inlets and two outlets as depicted in Fig. 1. In each of the filter modules, the sample being sorted is introduced from the container 10 or the chamber 50 through one of the inlets, and the liquid is introduced from the liquid container 40 through the other inlet. Thereafter, the sample is separated in each of the filter modules, and a mixture containing solids smaller in size flows out from one of the outlets, and a mixture containing solids larger in size flows out from the other outlet.

In a case where the sample being sorted is a blood-derived sample, and the target bioparticles are white blood cells, in the first filter module 20, red blood cells and platelets smaller than the white blood cells can be removed, and furthermore, the solvent can be replaced with the liquid from the liquid container 40. It is therefore possible to recover the white blood cells in high yields with neither waste nor damage to the white blood cells. Furthermore, in the second filter module 21, cells larger than lymphocytes can be removed.

In the sample sorting system of the present technology, selection of the target bioparticles using a label is performed, thereby allowing an increase in the concentration of the target bioparticles in the sample being sorted. As this selection, positive selection for sorting labeled particles obtained by labeling the target bioparticles, negative selection for removing labeled particles obtained by labeling particles other than the target bioparticles, and a combination thereof can be used. In the sample sorting system 1 depicted in Fig. 1, the sample being sorted containing the target bioparticles is introduced into the chamber 50, and a labeling reagent is introduced from the first reagent container 30 into the chamber 50, thereby allowing the target bioparticles or the particles other than the target bioparticles to be labeled. The separation of the labeled particles can be performed in accordance with the properties of the label.

In the negative selection, a plurality of particles (for example, lymphocytes and red blood cells, and the like) other than the target bioparticles may be labeled, and in this case, a plurality of kinds of labeling reagents can also be used. The plurality of kinds of labeling reagents may be separately stored in a plurality of reagent containers, or may be mixed in advance and stored in the first reagent container 30.

The labeling reagents used for the positive selection and the negative selection may be a conjugate of a labeling substance and a substance that traps the particles of interest. The substance that traps the particles of interest may be, for example, a substance that binds itself to the particles of interest (also referred to as a "particle-binding substance"), or a substance that traps the particles of interest with the help of another substance. In the latter case, the substance that traps the particles of interest itself is not a substance that binds to the particles, and the other substance may be a substance that binds to the target particles.

As described above, the particle-binding substance is a substance that binds itself to the particles of interest, and is, for example, an antibody or an antibody fragment. Specifically, the particle-binding substance may be an antibody or an antibody fragment that binds to an antigen present on the surfaces of the particles of interest, or may be, for example, an antibody or an antibody fragment that binds to an antigen present on the surfaces of cells.

As described above, the substance that traps the particles of interest with the help of the other substance itself need not necessarily be a substance that binds to the particles of interest. The substance that traps the particles of interest with the help of the other substance may be, for example, a substance that binds to the particle-binding substance such as a protein that binds to an antibody or an antibody fragment or a protein that specifically binds to an antibody or an antibody fragment. Examples of such a protein may include an antibody-binding protein, e.g., any one of Protein A, Protein G, Protein L, or Protein A/G, or any combination thereof.

The labeling substance contained in the labeling reagent used for the positive selection and the negative selection may be any labeling substance as long as the labeling substance enables separation of particles to which the label is bound, and more specifically, it is desirable that the labeling substance enables automatic separation of the particles to which the label is bound. As such a labeling substance, for example, beads can be suitably used. The beads are only required to have any size as long as the target bioparticles to which the beads are bound are separated as a mixture of solids larger in size than the target bioparticles in the second filter module, for example.

In a case where the beads are used as the label for the positive selection and the negative selection, separation of particles to which the label is bound can be performed using the second filter module. That is, in a case of the positive selection, since the beads are bound to the target bioparticles, it is possible to sort, by causing the sample being sorted to flow through the second filter module, the target bioparticles to which the beads are bound as a mixture of solids larger in size than the target bioparticles. In a case of the negative selection, since the beads are bound to particles other than the target bioparticles, it is possible to sort, by causing the sample being sorted to flow through the second filter module, the particles to which the beads are bound as a mixture of solids larger in size than the target bioparticles.

In a case where the negative selection is performed using the bead label, not only particles to which the beads are bound but also solids to which no beads are bound, the solids being larger in size than the target bioparticles, can be discarded as a mixture of solids larger in size than the target bioparticles by the second filter module. Therefore, in this case, the processing of removing large-sized solids by the second filter module 21 without using a label need not necessarily be performed.

Only either one or both of the positive selection and the negative selection may be performed. In a case where both the positive selection and the negative selection are performed, it is necessary to provide another reagent container installed at a position similar to the position of the first reagent container 30 in order to separately store necessary labeling reagents.

As described above, a target biological sample containing concentrated target bioparticles can be sorted from the sample being sorted by a combination of (a), (b), (c1) and (c2), a combination of (a), (c1) and (c2), a combination of (a), (b) and (c1), a combination of (a), (b) and (c2), or a combination of (a) and (c2), where:
(a) is the removal of small particles by the first filter module; (b) is the removal of unlabeled large particles by the second filter module; (c1) is the positive selection; and (c2) is the negative selection. Here, in a case where the bead label is used in the processing (c1) and the processing (c2), and the processing (b) and/or the processing (c2) is performed after the processing (c1), processing of removing the beads from the target bioparticles is performed after the processing (c1) and before the processing (b) and/or the processing (c2). Furthermore, in other cases as well, the processing of removing the beads from the target bioparticles may be performed.

### (vi) Fluorescence Staining

Moreover, the sample being sorted can be labeled (stained) with a fluorescent dye at any timing before the target biological sample is completed. In this case, the sample sorting system of the present technology may further include a labeling part that labels the sample being sorted with a fluorescent dye, and the labeling part may be airtightly connected to at least one of the container or the sorting part. For example, in the sample sorting system 1 depicted in Fig. 1, a fluorescent labeling reagent is stored in the third reagent container 32, and the fluorescent labeling reagent is mixed with the sample being sorted in the chamber 50, thereby allowing the sample being sorted to be labeled with the fluorescent dye. Furthermore, this processing of labeling with the fluorescent dye can be performed simultaneously with the labeling processing in the positive selection and/or the negative selection described above.

The type and number of fluorescent dyes used for the above-described staining are not particularly limited, and known dyes such as fluorescein isothiocyanete (FITC: C21H11NO5S), phycoerythrin (PE), periidinin chlorophyll protein (PerCP), PE-Cy5, and PE-Cy7 can be appropriately selected and used as necessary. Furthermore, the fluorescent dye may be bound to the sample being sorted with the help of a degradable linker, particularly a photodegradable linker. Furthermore, a fluorescently labeled antibody may be used as the fluorescent labeling reagent, which allows only the target bioparticles to be stained. Furthermore, a plurality of kinds of fluorescent labeling reagents can be blended in advance so as to adapt to staining with multiple markers (multiple colors). The fluorescent labeling reagent may be in a solution state or in a freeze-dried state.

### (vii) Concentration Adjustment

Moreover, before the target biological sample is completed, the concentration of particles suspended in a liquid in the sample being sorted can be measured, and the concentration can be adjusted as necessary. A method for measuring the concentration of particles such as cells may be any method as long as it is possible to measure the concentration of particles in a sample without touching the sample in a sealed flow channel, and for example, an optical method, particularly, a turbidity measurement method or the like is suitably used. For example, in the sample sorting system 1 depicted in Fig. 1, the concentration sensor 80 is provided upstream of the storage part 60, and a flow channel and a valve are arranged so as to allow the sample flowing out from the concentration sensor 80 to flow into both the storage part 60 and the chamber 50.

In a case where a result of the concentration measurement shows that the concentration of the particles in the sample being sorted falls within an appropriate range, the sample can be stored in the storage part as the target biological sample. On the other hand, in a case where it is determined that the concentration of the particles in the sample being sorted falls outside the appropriate range, the sample can be concentrated or diluted.

The sample being sorted can be diluted by mixing the sample being sorted and the liquid from the first liquid container 40 at an appropriate ratio in the chamber 50.

The sample being sorted can be concentrated by means of, for example, a filter module. In the filter module such as a microchannel, when the particles are separated and guided to two outlets in accordance with their respective sizes, the liquid from the liquid container 40 becomes different in flow rate between the two outlets, thereby making the concentration of the particles in the sample flowing out from the outlets different. It is therefore possible to concentrate the sample being sorted containing the target bioparticles by causing the sample being sorted to flow through a filter module that separates and guides the target bioparticles to an outlet lower in liquid flow rate, of the first filter module and the second filter module. In many filter modules, the outlet to which larger-sized particles are guided is lower in liquid flow rate, so that the concentration of the particles becomes higher. It is therefore possible to concentrate of the sample being sorted, by, for example, causing the sample being sorted to flow through the first filter module to cause the sample being sorted containing the target bioparticles to flow out from the outlet through which large-sized particles flows out.

In a case where a microchannel is used as the filter module, it is possible to move the particles of interest (cells or the like) to, for example, a curved channel to merge the particles of interest into a narrow flow by fluid lift. It is therefore possible to concentrate the particles of interest by discarding the flow of a liquid (such as a buffer solution) not containing the particles of interest to reduce the amount of a liquid in the flow containing the particles of interest.

Alternatively, it is possible to concentrate the sample being sorted using a third filter module different from the first filter module and the second filter module described above. As such a third filter module, it is possible to use a filter module having an outlet to which small-size particles are guided and an outlet to which large-size particles are guided, the outlet higher in liquid flow rate (that is, the flow rate of the liquid from the liquid container 40 is higher) being opposite to the first filter module or the second filter module. The use of such a filter module as necessary instead of the first filter module or the second filter module can prevent the sample being sorted from diluting. For example, in a case where the second filter module is of a type in which the outlet to which small-sized particles are guided is higher in liquid flow rate, the use, as the third filter module, of a filter module of a type in which the outlet to which large-sized particles are guided on the basis of the same threshold is higher in liquid flow rate and the appropriate use of the filter module instead of the second filter module allow the concentrated sample being sorted containing the target bioparticles to flow out from the outlet to which small-sized particles are guided.

Alternatively, as the third filter module, a filter module that has an outlet to which small-size particles are guided and an outlet to which large-size particles are guided, and causes the sample being sorted containing the target bioparticles to flow out from the outlet opposite to the outlet that is higher in liquid flow rate (that is, the flow rate of the liquid from the liquid container 40 is higher). Causing the sample being sorted containing the target bioparticles flowing out from the outlet of the first filter module or the second filter module to flow through such a third filter module allows the concentrated sample being sorted containing the target bioparticles to flow out.

The sample being sorted may be concentrated before the concentration measurement, and in this case, the result of the concentration measurement shows that the concentration falls within the appropriate range or higher than the appropriate range. In a case where the result shows that the concentration falls within the appropriate range, the sample being sorted can be stored in the storage part as the target biological sample, and in a case where the result shows that the concentration is higher than the appropriate range, the sample can be diluted.

### (viii) Dead Volume Recovery Processing

Moreover, in the sample sorting system of the present technology, even after the sorting processing is completed, the target bioparticles may remain in a place other than a place where the sample being sorted containing the target bioparticles exists, for example, the container, the first and second filter modules, the chamber, the flow channel, and the like, and the amount of remaining target bioparticles is defined as a dead volume. In the sample sorting system of the present technology, the target biological sample containing as many target bioparticles as possible can be obtained by recovering the dead volume.

The dead volume can be recovered, for example, by causing the liquid to flow from the liquid container 40 to the container and a place where the sample being sorted has flowed through to cause the liquid that has flowed through to merge with the sample being sorted. Here, in a case where the sample contained in the liquid that has been caused to flow through (liquid to merge with the sample being sorted) has not undergone the processing of concentrating the target bioparticles in the sorting part, it is desirable that the processing of concentrating the target bioparticles in the sorting part be performed on the liquid before merging with the sample being sorted. Furthermore, the processing of concentrating the target bioparticles in the sorting part as described above may be performed on a mixture obtained as a result of merging the liquid that has flowed through with the sample being sorted. In particular, the processing of recovering the dead volume as described above may be performed on the container or a place where the target bioparticles are likely to remain after the sample flows through, for example, a filter module.

### <2. Specific Example of Sample Sorting by Sample Sorting System>

Hereinafter, sample sorting by the sample sorting system will be described with reference to a specific example. In the following specific example, a target biological sample containing concentrated white blood cells (target bioparticles) is obtained from a blood-derived sample (sample being sorted). In the following specific example, a chip A is a microchannel chip as the first filter module, and a mixture containing white blood cells is obtained from a large cell outlet provided in the chip, and a mixture of solids smaller in size than white blood cells is obtained from a small cell outlet.

A chip B is a microchannel chip as the second filter module, and a mixture of solids larger in size than white blood cells is obtained from a large cell outlet provided in the chip, and a mixture containing white blood cells is obtained from a small cell outlet. In either of the chips, the solvent of the sample flowing out from the large cell outlet decreases, and the solvent of the sample flowing out from the small cell outlet increases.

### (i) Specific Example of Sample Sorting Using Negative Selection

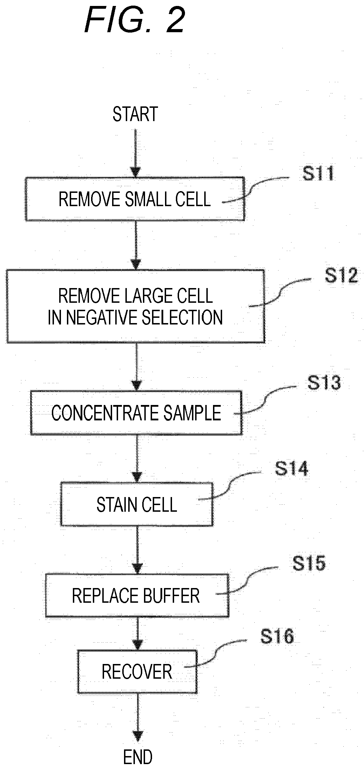

The outline will be described in the flowchart in Fig. 2. Specific processes will be described in Table 1 below.

**[Table 1]**

| [Table 1] IN A CASE WHERE NEGATIVE SELECTION IS PERFORMED WITH SPECIFIC CONCENTRATION | | |
|---|---|---|
| PROCESS No. | OPERATION DETAILS | OPERATION IN Fig. 1 AND REMARKS |
| 1 | REMOVAL OF RED BLOOD CELL (S11 IN Fig. 2) | MOVE SAMPLE FROM CONTAINER 10 TO CHIP A20 AND MOVE PBS(+) FROM LIQUID CONTAINER 40 TO CHIP A20 AT THE SAME TIME. MOVE SAMPLE FROM LARGE CELL OUTLET OF CHIP A20 TO CHAMBER 50. MOVE SAMPLE FROM SMALL CELL OUTLET OF CHIP A20 TO DISPOSAL PART 70. |
| 2 | SAMPLE BACK RINSE | TO USE INPUT SAMPLE WITHOUT WASTE, MOVE PBS(+) FROM LIQUID CONTAINER 40 TO CONTAINER 10. |
| 3 | REMOVAL OF RED BLOOD CELL FROM RINSE SAMPLE | SAME AS PROCESS 1. |
| 4 | DILUTE SAMPLE IN CHAMBER (IF NECESSARY) | IN A CASE WHERE CONCENTRATION OF SAMPLE IN CHAMBER IS HIGHER THAN ALLOWABLE CONCENTRATION, MOVE PBS(+) FROM LIQUID CONTAINER 40 TO CHAMBER 50. |
| 5 | CLEANING OF CHIP A | CAUSE CLEANING LIQUID TO FLOW FROM LIQUID CONTAINER 41 TO TWO INLETS OF CHIP A20 THROUGH pumpA AND pumpB AND MOVE LIQUID FROM BOTH TWO OUTLETS OF CHIP A20 TO DISPOSAL PART 70. AT THIS TIME, IT IS PREFERABLE THAT pumpA BE HIGHER IN FLOW RATE THAN pumpB. IF NECESSARY, PBS(+) MAY BE SIMILARLY CAUSED TO FLOW FROM LIQUID CONTAINER 40 FOR RINSE. |
| 6 | INPUT OF NEGATIVE SELECTION REAGENT (S12 IN Fig. 2) | MOVE LABELING REAGENT USED TO BIND BEADS TO CELLS OTHER THAN WHITE BLOOD CELLS FROM REAGENT CONTAINER 30 TO CHAMBER 50 THROUGH pumpC. |
| 7 | CIRCULATION FOR AGITATION (IF NECESSARY) | REPEATEDLY MOVE, WITH pumpC, MIXTURE OF SAMPLE AND LABELING REAGENT FROM CHAMBER 50 THROUGH CONCENTRATION SENSOR 80 AND RETURN MIXTURE TO CHAMBER 50. IT IS PREFERABLE THAT MIXTURE BE CIRCULATED MILDLY AT LOW FLOW RATE. |
| 8 | SAMPLE RETURN | MOVE SAMPLE FROM CHAMBER 50 TO CONTAINER 10. CONTAINER 11 ONLY USED FOR SAMPLE TO RETURN MAY BE ADDITIONALLY PROVIDED. |
| 9 | RINSE OF CHAMBER AND CIRCULATION CIRCUIT | CAUSE PBS(+) TO FLOW FROM LIQUID CONTAINER 40 THROUGH CHIP A20, CHAMBER 50, AND CONCENTRATION SENSOR 80. MOVE PBS(+) THAT HAS FLOWED THROUGH CHIP A20 TO DISPOSAL PART 70. MOVE PBS(+) THAT HAS FLOWED THROUGH CONCENTRATION SENSOR 80 TO CHAMBER 50. AT THIS TIME, IT IS PREFERABLE THAT FLOW RATE SATISFY PumpA > B > C. |
| 10 | RINSE SAMPLE RETURN | MOVE LIQUID FROM CHAMBER 50 TO CONTAINER 10 AND MERGE LIQUID WITH SAMPLE RETURNED PREVIOUSLY. CONTAINER 11 ONLY USED FOR SAMPLE TO RETURN MAY BE ADDITIONALLY PROVIDED. |
| 11 | REMOVAL OF LARGE CELL AND NEGATIVE SELECTION | MOVE SAMPLE FROM CONTAINER 10 TO CHIP B21 AND MOVE PBS(+) FROM LIQUID CONTAINER 40 TO CHIP B21 AT THE SAME TIME. MOVE SAMPLE FROM SMALL CELL OUTLET OF CHIP B21 TO CHAMBER 50. MOVE SAMPLE FROM LARGE CELL OUTLET OF CHIP B21 TO DISPOSAL PART 70. |
| 12 | SAMPLE BACK RINSE | TO USE INPUT SAMPLE WITHOUT WASTE, MOVE PBS(+) FROM LIQUID CONTAINER 40 TO CONTAINER 10. |
| 13 | REMOVAL OF LARGE CELL OF RINCE REAGENT AND NEGATIVE SELECTION | SAME AS PROCESS 11. |
| 14 | CLEANING OF CHIP B | CAUSE CLEANING LIQUID TO FLOW FROM LIQUID CONTAINER 41 TO TWO INLETS OF CHIP B21 THROUGH pumpA AND pumpB AND MOVE LIQUID FROM BOTH TWO OUTLETS OF CHIP B21 TO DISPOSAL PART 70. AT THIS TIME, IT IS PREFERABLE THAT pumpA BE HIGHER IN FLOW RATE THAN pumpB. IF NECESSARY, PBS(+) MAY BE SIMILARLY CAUSED TO FLOW FROM LIQUID CONTAINER 40 FOR RINSE. |
| 15 | SAMPLE RETURN | MOVE SAMPLE FROM CHAMBER 50 TO CONTAINER 10. CONTAINER 11 ONLY USED FOR SAMPLE TO RETURN MAY BE ADDITIONALLY PROVIDED. |
| 16 | RINSE OF CHAMBER AND CIRCULATION CIRCUIT (IF NECESSARY) | CAUSE PBS(+) TO FLOW FROM LIQUID CONTAINER 40 THROUGH CHIP B21, CHAMBER 50, AND CONCENTRATION SENSOR 80. MOVE PBS(+) THAT HAS FLOWED THROUGH CHIP B21 TO DISPOSAL PART 70. MOVE PBS(+) THAT HAS FLOWED THROUGH CONCENTRATION SENSOR 80 TO CHAMBER 50. AT THIS TIME, IT IS PREFERABLE THAT FLOW RATE SATISFY PumpA > B > C. |
| 17 | RINSE SAMPLE RETURN | MOVE LIQUID FROM CHAMBER 50 TO CONTAINER 10 AND MERGE LIQUID WITH SAMPLE RETURNED PREVIOUSLY. CONTAINER 11 ONLY USED FOR SAMPLE TO RETURN MAY BE ADDITIONALLY PROVIDED. |
| 18 | INCREASE OF CONCENTRATION OF SAMPLE DILUTED IN NEGATIVE SELECTION (S13 IN Fig. 2) | MOVE SAMPLE FROM CONTAINER 10 TO CHIP A20 AND MOVE PBS(+) FROM LIQUID CONTAINER 40 TO CHIP A20 AT THE SAME TIME. MOVE SAMPLE FROM LARGE CELL OUTLET OF CHIP A20 TO CHAMBER 50. MOVE SAMPLE FROM SMALL CELL OUTLET OF CHIP A20 TO DISPOSAL PART 70. |
| 19 | SAMPLE BACK RINSE | TO USE INPUT SAMPLE WITHOUT WASTE, MOVE PBS(+) FROM LIQUID CONTAINER 40 TO CONTAINER 10. |
| 20 | PROCESSING ON SAMPLE AFTER RINSE | SAME AS PROCESS 18. IT IS PREFERABLE THAT AMOUNT OF RINSE LIQUID IN PROCESS 19 BE ADJUSTED IN ORDER TO MAKE CONCENTRATION OF SAMPLE IN CHAMBER 50 SUITABLE FOR SUBSEQUENT STAINING PROCESS. IN A CASE WHERE INCREASE OF CONCENTRATION IS INSUFFICIENT, IT IS PREFERABLE THAT PROCESSES 15 TO 20 BE REPEATED. |
| 21 | CLEANING OF CHIP A | CAUSE CLEANING LIQUID TO FLOW FROM LIQUID CONTAINER 41 TO TWO INLETS OF CHIP A20 THROUGH pumpA AND pumpB AND MOVE LIQUID FROM BOTH TWO OUTLETS OF CHIP A20 TO DISPOSAL PART 70. AT THIS TIME, IT IS PREFERABLE THAT pumpA BE HIGHER IN FLOW RATE THAN pumpB. IF NECESSARY, PBS(+) MAY BE SIMILARLY CAUSED TO FLOW FROM LIQUID CONTAINER 40 FOR RINSE. |
| 22 | INPUT OF STAINING REAGENT (S14 IN Fig. 2) | MOVE STAINING REAGENT FROM REAGENT CONTAINER 32 TO CHAMBER 50 THROUGH pumpC. |
| 23 | CIRCULATION FOR AGITATION (IF NECESSARY) | REPEATEDLY MOVE, WITH pumpC, MIXTURE OF SAMPLE AND STAINING REAGENT FROM CHAMBER 50 THROUGH CONCENTRATION SENSOR 80 AND RETURN MIXTURE TO CHAMBER 50. IT IS PREFERABLE THAT MIXTURE BE CIRCULATED MILDLY AT LOW FLOW RATE. |
| 24 | SAMPLE RETURN | MOVE SAMPLE FROM CHAMBER 50 TO CONTAINER 10. CONTAINER 11 ONLY USED FOR SAMPLE TO RETURN MAY BE ADDITIONALLY PROVIDED. |
| 25 | RINSE OF CHAMBER AND CIRCULATION CIRCUIT | CAUSE PBS(+) TO FLOW FROM LIQUID CONTAINER 40 THROUGH CHAMBER 50, AND CONCENTRATION SENSOR 80. MOVE PBS(+) THAT HAS FLOWED THROUGH CONCENTRATION SENSOR 80 TO CHAMBER 50. AT THIS TIME, IT IS PREFERABLE THAT FLOW RATE SATISFY PumpA > B > C. |
| 26 | RINSE REAGENT RETURN | MOVE LIQUID FROM CHAMBER 50 TO CONTAINER 10 AND MERGE LIQUID WITH SAMPLE RETURNED PREVIOUSLY. CONTAINER 11 ONLY USED FOR SAMPLE TO RETURN MAY BE ADDITIONALLY PROVIDED. |
| 27 | CLEANING (REPLACEMENT OF BUFFER) (S15 IN Fig. 2) | MOVE SAMPLE FROM CONTAINER 10 TO CHIP A20 AND MOVE BUFFER SUITABLE FOR SUBSEQUENT PROCESSING FROM ADDITIONALLY PROVIDED LIQUID CONTAINER TO CHIP A20 AT THE SAME TIME. MOVE SAMPLE FROM LARGE CELL OUTLET OF CHIP A20 TO CHAMBER 50. MOVE SAMPLE FROM SMALL CELL OUTLET OF CHIP A20 TO DISPOSAL PART 70. |
| 28 | SAMPLE BACK RINSE | TO USE INPUT SAMPLE WITHOUT WASTE, MOVE BUFFER SUITABLE FOR SUBSEQUENT PROCESSING FROM ADDITIONALLY PROVIDED LIQUID CONTAINER TO CONTAINER 10. |
| 29 | PROCESSING ON SAMPLE AFTER RINSE | SAME AS PROCESS 27. |
| 30 | RECOVERY (S16 IN Fig. 2) | MOVE, WITH pumpC, SAMPLE IN CHAMBER 50 TO STORAGE PART 60. |
| 31 | RINSE OF CHAMBER AND CIRCULATION CIRCUIT | CAUSE BUFFER SUITABLE FOR SUBSEQUENT PROCESSING TO FLOW FROM ADDITIONALLY PROVIDED LIQUID CONTAINER THROUGH CHAMBER 50, AND CONCENTRATION SENSOR 80. MOVE BUFFER THAT HAS FLOWED THROUGH CONCENTRATION SENSOR 80 TO CHAMBER 50. AT THIS TIME, IT IS PREFERABLE THAT FLOW RATE SATISFY PumpA > B > C. AMOUNT OF BUFFER USED FOR RINSE IS ADJUSTED ON THE BASIS OF INTENDED CELL CONCENTRATION OF FINAL RECOVERED OBJECT. |
| 32 | ADDITIONAL RECOVERY AFTER RINSE | MOVE, WITH pumpC, SAMPLE IN CHAMBER 50 TO STORAGE PART 60. |

### (ii) Specific Example of Sample Sorting Using Positive Selection

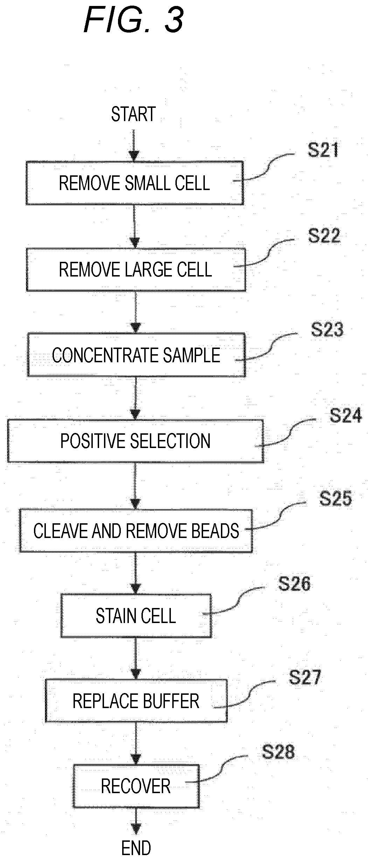

The outline will be described in the flowchart in Fig. 3. Specific processes will be described in Table 2 below.

**[Table 2]**

| [Table 2] IN A CASE WHERE POSITIVE SELECTION IS PERFORMED WITH SPECIFIC CONCENTRATION | | |
|---|---|---|
| PROCESS No. | OPERATION DETAILS | OPERATION IN Fig. 1 AND REMARKS |
| 1 | REMOVAL OF RED BLOOD CELL (S11 IN Fig. 2) | MOVE SAMPLE FROM CONTAINER 10 TO CHIP A20 AND MOVE PBS(+) FROM LIQUID CONTAINER 40 TO CHIP A20 AT THE SAME TIME. MOVE SAMPLE FROM LARGE CELL OUTLET OF CHIP A20 TO CHAMBER 50. MOVE SAMPLE FROM SMALL CELL OUTLET OF CHIP A20 TO DISPOSAL PART 70. |
| 2 | SAMPLE BACK RINSE | TO USE INPUT SAMPLE WITHOUT WASTE, MOVE PBS(+) FROM LIQUID CONTAINER 40 TO CONTAINER 10. |
| 3 | REMOVAL OF RED BLOOD CELL FROM RINSE SAMPLE | SAME AS PROCESS 1. |
| 4 | DILUTE SAMPLE IN CHAMBER (IF NECESSARY) | IN A CASE WHERE CONCENTRATION OF SAMPLE IN CHAMBER IS HIGHER THAN ALLOWABLE CONCENTRATION, MOVE PBS(+) FROM LIQUID CONTAINER 40 TO CHAMBER 50. |
| 5 | CLEANING OF CHIP A | CAUSE CLEANING LIQUID TO FLOW FROM LIQUID CONTAINER 41 TO TWO INLETS OF CHIP A20 THROUGH pumpA AND pumpB AND MOVE LIQUID FROM BOTH TWO OUTLETS OF CHIP A20 TO DISPOSAL PART 70. AT THIS TIME, IT IS PREFERABLE THAT pumpA BE HIGHER IN FLOW RATE THAN pumpB. IF NECESSARY, PBS(+) MAY BE SIMILARLY CAUSED TO FLOW FROM LIQUID CONTAINER 40 FOR RINSE. |
| 6 | SAMPLE RETURN | MOVE SAMPLE FROM CHAMBER 50 TO CONTAINER 10. CONTAINER 11 ONLY USED FOR SAMPLE TO RETURN MAY BE ADDITIONALLY PROVIDED. |
| 7 | RINSE OF CHAMBER AND CIRCULATION CIRCUIT | CAUSE PBS(+) TO FLOW FROM LIQUID CONTAINER 40 THROUGH CHIP A20, CHAMBER 50, AND CONCENTRATION SENSOR 80. MOVE PBS(+) THAT HAS FLOWED THROUGH CHIP A20 TO DISPOSAL PART 70. MOVE PBS(+) THAT HAS FLOWED THROUGH CONCENTRATION SENSOR 80 TO CHAMBER 50. AT THIS TIME, IT IS PREFERABLE THAT FLOW RATE SATISFY PumpA > B > C. |
| 8 | RINSE SAMPLE RETURN | MOVE LIQUID FROM CHAMBER 50 TO CONTAINER 10 AND MERGE LIQUID WITH SAMPLE RETURNED PREVIOUSLY. CONTAINER 11 ONLY USED FOR SAMPLE TO RETURN MAY BE ADDITIONALLY PROVIDED. |
| 9 | REMOVAL OF LARGE CELL (S22 IN Fig. 3) | MOVE SAMPLE FROM CONTAINER 10 TO CHIP B21 AND MOVE PBS(+) FROM LIQUID CONTAINER 40 TO CHIP B21 AT THE SAME TIME. MOVE SAMPLE FROM SMALL CELL OUTLET OF CHIP B21 TO CHAMBER 50. MOVE SAMPLE FROM LARGE CELL OUTLET OF CHIP B21 TO DISPOSAL PART 70. |
| 10 | SAMPLE BACK RINSE | TO USE INPUT SAMPLE WITHOUT WASTE, MOVE PBS(+) FROM LIQUID CONTAINER 40 TO CONTAINER 10. |
| 11 | REMOVAL OF LARGE CELL OF RINCE REAGENT | SAME AS PROCESS 9. |
| 12 | CLEANING OF CHIP B | CAUSE CLEANING LIQUID TO FLOW FROM LIQUID CONTAINER 41 TO TWO INLETS OF CHIP B21 THROUGH pumpA AND pumpB AND MOVE LIQUID FROM BOTH TWO OUTLETS OF CHIP B21 TO DISPOSAL PART 70. AT THIS TIME, IT IS PREFERABLE THAT pumpA BE HIGHER IN FLOW RATE THAN pumpB. IF NECESSARY, PBS(+) MAY BE SIMILARLY CAUSED TO FLOW FROM LIQUID CONTAINER 40 FOR RINSE. |
| 13 | SAMPLE RETURN | MOVE SAMPLE FROM CHAMBER 50 TO CONTAINER 10. CONTAINER 11 ONLY USED FOR SAMPLE TO RETURN MAY BE ADDITIONALLY PROVIDED. |
| 14 | RINSE OF CHAMBER AND CIRCULATION CIRCUIT (IF NECESSARY) | CAUSE PBS(+) TO FLOW FROM LIQUID CONTAINER 40 THROUGH CHIP B21, CHAMBER 50, AND CONCENTRATION SENSOR 80. MOVE PBS(+) THAT HAS FLOWED THROUGH CHIP B21 TO DISPOSAL PART 70. MOVE PBS(+) THAT HAS FLOWED THROUGH CONCENTRATION SENSOR 80 TO CHAMBER 50. AT THIS TIME, IT IS PREFERABLE THAT FLOW RATE SATISFY PumpA > B > C. |
| 15 | RINSE SAMPLE RETURN | MOVE LIQUID FROM CHAMBER 50 TO CONTAINER 10 AND MERGE LIQUID WITH SAMPLE RETURNED PREVIOUSLY. CONTAINER 11 ONLY USED FOR SAMPLE TO RETURN MAY BE ADDITIONALLY PROVIDED. |
| 16 | INCREASE OF CONCENTRATION OF SAMPLE DILUTED IN PROCESS OF REMOVING LARGE CELL (S23 IN Fig. 3) | MOVE SAMPLE FROM CONTAINER 10 TO CHIP A20 AND MOVE PBS(+) FROM LIQUID CONTAINER 40 TO CHIP A20 AT THE SAME TIME. MOVE SAMPLE FROM LARGE CELL OUTLET OF CHIP A20 TO CHAMBER 50. MOVE SAMPLE FROM SMALL CELL OUTLET OF CHIP A20 TO DISPOSAL PART 70. |
| 17 | SAMPLE BACK RINSE | TO USE INPUT SAMPLE WITHOUT WASTE, MOVE PBS(+) FROM LIQUID CONTAINER 40 TO CONTAINER 10. |
| 18 | PROCESSING ON SAMPLE AFTER RINSE | SAME AS PROCESS 16. IT IS PREFERABLE THAT AMOUNT OF RINSE LIQUID IN PROCESS 17 BE ADJUSTED IN ORDER TO MAKE CONCENTRATION OF SAMPLE IN CHAMBER 50 SUITABLE FOR SUBSEQUENT PROCESS. IN A CASE WHERE INCREASE OF CONCENTRATION IS INSUFFICIENT, IT IS PREFERABLE THAT PROCESSES 16 TO 18 BE REPEATED. |
| 19 | CLEANING OF CHIP A | CAUSE CLEANING LIQUID TO FLOW FROM LIQUID CONTAINER 41 TO TWO INLETS OF CHIP A20 THROUGH pumpA AND pumpB AND MOVE LIQUID FROM BOTH TWO OUTLETS OF CHIP A20 TO DISPOSAL PART 70. AT THIS TIME, IT IS PREFERABLE THAT pumpA BE HIGHER IN FLOW RATE THAN pumpB. IF NECESSARY, PBS(+) MAY BE SIMILARLY CAUSED TO FLOW FROM LIQUID CONTAINER 40 FOR RINSE. |
| 20 | INPUT OF POSITIVE SELECTION REAGENT (S24 IN Fig. 3) | MOVE LABELING REAGENT USED TO BIND BEADS TO WHITE BLOOD CELLS FROM REAGENT CONTAINER 30 TO CHAMBER 50 THROUGH pumpC. |
| 21 | CIRCULATION FOR AGITATION (IF NECESSARY) | REPEATEDLY MOVE, WITH pumpC, MIXTURE OF SAMPLE AND LABELING REAGENT FROM CHAMBER 50 THROUGH CONCENTRATION SENSOR 80 AND RETURN MIXTURE TO CHAMBER 50. IT IS PREFERABLE THAT MIXTURE BE CIRCULATED MILDLY AT LOW FLOW RATE. |
| 22 | SAMPLE RETURN | MOVE SAMPLE FROM CHAMBER 50 TO CONTAINER 10. CONTAINER 11 ONLY USED FOR SAMPLE TO RETURN MAY BE ADDITIONALLY PROVIDED. |
| 23 | RINSE OF CHAMBER AND CIRCULATION CIRCUIT (IF NECESSARY) | CAUSE PBS(+) TO FLOW FROM LIQUID CONTAINER 40 THROUGH CHIP A20, CHAMBER 50, AND CONCENTRATION SENSOR 80. MOVE PBS(+) THAT HAS FLOWED THROUGH CHIP A20 TO DISPOSAL PART 70. MOVE PBS(+) THAT HAS FLOWED THROUGH CONCENTRATION SENSOR 80 TO CHAMBER 50. AT THIS TIME, IT IS PREFERABLE THAT FLOW RATE SATISFY PumpA > B > C. |
| 24 | RINSE REAGENT RETURN | MOVE LIQUID FROM CHAMBER 50 TO CONTAINER 10 AND MERGE LIQUID WITH SAMPLE RETURNED PREVIOUSLY. CONTAINER 11 ONLY USED FOR SAMPLE TO RETURN MAY BE ADDITIONALLY PROVIDED. |
| 25 | POSITIVE SELECTION | MOVE SAMPLE FROM CONTAINER 10 TO CHIP B21 AND MOVE PBS(+) FROM LIQUID CONTAINER 40 TO CHIP B21 AT THE SAME TIME. MOVE SAMPLE FROM SMALL CELL OUTLET OF CHIP B21 TO DISPOSAL PART 70. MOVE SAMPLE FROM LARGE CELL OUTLET OF CHIP B21 TO CHAMBER 50. |
| 26 | SAMPLE BACK RINSE | TO USE INPUT SAMPLE WITHOUT WASTE, MOVE PBS(+) FROM LIQUID CONTAINER 40 TO CONTAINER 10. |
| 27 | POSITIVE SELECTION OF RINSE SAMPLE | SAME AS PROCESS 25. |
| 28 | CLEANING OF CHIP B | CAUSE CLEANING LIQUID TO FLOW FROM LIQUID CONTAINER 41 TO TWO INLETS OF CHIP B21 THROUGH pumpA AND pumpB AND MOVE LIQUID FROM BOTH TWO OUTLETS OF CHIP B21 TO DISPOSAL PART 70. AT THIS TIME, IT IS PREFERABLE THAT pumpA BE HIGHER IN FLOW RATE THAN pumpB. IF NECESSARY, PBS(+) MAY BE SIMILARLY CAUSED TO FLOW FROM LIQUID CONTAINER 40 FOR RINSE. |
| 29 | MIXING WITH REAGENT USED TO CLEAVE POSITIVE SELECTION BEADS (S25 IN Fig. 3) | MOVE REAGENT USED TO CLEAVE BEADS FROM ADDITIONALLY PROVIDED REAGENT CONTAINER TO CHAMBER 50 THROUGH pumpC. |
| 30 | CIRCULATION FOR AGITATION (IF NECESSARY) | REPEATEDLY MOVE, WITH pumpC, MIXTURE OF SAMPLE AND CLEAVING REAGENT FROM CHAMBER 50 THROUGH CONCENTRATION SENSOR 80 AND RETURN MIXTURE TO CHAMBER 50. IT IS PREFERABLE THAT MIXTURE BE CIRCULATED MILDLY AT LOW FLOW RATE. |
| 31 | SAMPLE RETURN | MOVE SAMPLE FROM CHAMBER 50 TO CONTAINER 10. CONTAINER 11 ONLY USED FOR SAMPLE TO RETURN MAY BE ADDITIONALLY PROVIDED. |
| 32 | RINSE OF CHAMBER AND CIRCULATION CIRCUIT (IF NECESSARY) | CAUSE PBS(+) TO FLOW FROM LIQUID CONTAINER 40 THROUGH CHIP B21, CHAMBER 50, AND CONCENTRATION SENSOR 80. MOVE PBS(+) THAT HAS FLOWED THROUGH CHIP B21 TO DISPOSAL PART 70. MOVE PBS(+) THAT HAS FLOWED THROUGH CONCENTRATION SENSOR 80 TO CHAMBER 50. AT THIS TIME, IT IS PREFERABLE THAT FLOW RATE SATISFY PumpA > B > C. |
| 33 | RINSE REAGENT RETURN | MOVE LIQUID FROM CHAMBER 50 TO CONTAINER 10 AND MERGE LIQUID WITH SAMPLE RETURNED PREVIOUSLY. CONTAINER 11 ONLY USED FOR SAMPLE TO RETURN MAY BE ADDITIONALLY PROVIDED. |
| 34 | REMOVAL OF CLEAVED BEADS | IN A CASE WHERE BEADS LARGER THAN WHITE BLOOD CELLS ARE USED, SAME AS PROCESSES 9 TO 19. IN A CASE WHERE BEADS SMALLER THAN WHITE BLOOD CELLS ARE USED, SAME AS PROCESSES 1 TO 5. |
| 35 | STAINING AND RECOVERY (S26 TO S28 IN Fig. 3) | SAME AS PROCESS 22 AND SUBSEQUENT PROCESSES IN Table 1. |

| | | |
|---|---|---|
| * 1 IF REACTION FOR POSITIVE SELECTION IS POSSIBLE EVEN WITHOUT INCREASE OF CONCENTRATION OF SAMPLE DILUTED IN PROCESS OF REMOVING LARGE CELL, PROCESSES 16 TO 19 NEED NOT BE PERFORMED. * 2 IN A CASE WHERE POSITIVE SELECTION IS PERFORMED A PLURALITY OF TIMES, PROCESSES 20 TO 34 OR PROCESSES 29 TO 34 ARE REPEATED BETWEEN PROCESS 34 AND PROCESS 35. | | |

### (iii) Specific Example of Sample Sorting Using Positive Selection and Negative Selection

Specific processes will be described in Table 3 below.

**[Table 3]**

| [Table 3] IN A CASE WHERE BOTH NEGATIVE SELECTION AND POSITIVE SELECTION ARE PERFORMED WITH SPECIFIC CONCENTRATION | | |
|---|---|---|
| PROCESS No. | OPERATION DETAILS | OPERATION IN Fig. 1 AND REMARKS |
| 1 | REMOVAL OF RED BLOOD CELL | SAME AS PROCESSES 1 TO 5 IN Table 1. |
| 2 | NEGATIVE SELECTION | SAME AS PROCESSES 6 TO 21 IN Table 1. |
| 3 | POSITIVE SELECTION | SAME AS PROCESSES 20 TO 34 IN Table 2. |
| 4 | STAINING AND RECOVERY | SAME AS PROCESS 22 AND SUBSEQUENT PROCESSES IN Table 1. |

### <3. Sample Sorting Device>

The present technology also provides a sample sorting device using the sample sorting system. The sample sorting device may primarily include the sample sorting system, a light irradiation unit, a light detection unit, and an arithmetic processing unit, and may include a position control unit, a degradation light irradiation unit, a drug feed management unit, a culture unit, and a pressure adjustment unit as necessary. Each of the units will be described below.

### (i) Sample Sorting System

The sample sorting device according to the present technology includes a sample sorting system that sorts and stores the target biological sample. The sample sorting system is the same in configuration as the sample sorting system 1 depicted in Fig. 1, so that no description will be given of the configuration.

### (ii) Light Irradiation Unit

The sample sorting device according to the present technology includes the light irradiation unit that irradiates the sample being sorted with light. The light irradiation unit includes, for example, a light source that emits excitation light, an objective lens that concentrates the excitation light onto the sample being sorted, and the like. The light source may be appropriately selected from a laser diode, a SHG laser, a solid-state laser, a gas laser, a high-luminance LED, and the like according to a purpose of analysis.
Furthermore, the light irradiation unit may include an optical element other than the light source and the objective lens as necessary.

### (iii) Light Detection Unit

The sample sorting device according to the present technology includes the light detection unit that detects fluorescence and scattered light emitted from the sample being sorted irradiated with excitation light. Specifically, the light detection unit detects the fluorescence and scattered light emitted from the sample being sorted and converts the same into an electric signal. Then, the electric signal is output to the arithmetic processing unit. The configuration of the light detection unit is not particularly limited, and a known configuration may be employed, and further, a method of converting into the electric signal is not particularly limited.

### (iv) Arithmetic Processing Unit

The sample sorting device according to the present technology includes the arithmetic processing unit to which the electric signal obtained by the conversion by the light detection unit is input. The arithmetic processing unit determines optical characteristics of the sample being sorted and the target bioparticles contained in the sample being sorted on the basis of the input electric signal. The arithmetic processing unit further includes a gating circuit that calculates a threshold used to sort the target bioparticles from the sample being sorted, a threshold used to determine whether or not the target bioparticles greater than or equal to the required number have been sorted, a threshold used to selectively separate the target bioparticles on the basis of intensity of fluorescence of a fluorescent dye used for labeling by the labeling part, and the like. With such a configuration of the gating circuit, in a case where the threshold used to sort the target biological sample from the sample being sorted is calculated, the threshold is converted into a sorting electric signal, and the sorting signal is output to a first pressure adjustment unit included in the sorting part. Note that the configuration of the arithmetic processing unit is not particularly limited, and a known configuration may be employed. Moreover, a known method may also be employed as an arithmetic processing method performed by the gating circuit of the arithmetic processing unit.

### (v) Position Control Unit

The sample sorting device according to the present technology may include the position control unit that controls the position of the light irradiation unit as necessary. The configuration of the position control unit is not particularly limited, and a known configuration may be employed. Examples of the position control unit include an actuator serving as a drive source.

### (vi) Degradation Light Irradiation Unit

The sample sorting device according to the present technology may include the degradation light irradiation unit as necessary. In a case where the sample sorting system includes the labeling part, and the sample being sorted is labeled with a fluorescent dye with the help of a photodegradable linker, it is necessary to remove the fluorescent dye from the sample being sorted in accordance with a use environment. The degradation light irradiation unit irradiates the photodegradable linker with predetermined light. As a result, the fluorescent dye can be removed from the sample being sorted. Here, a wavelength of the light with which the degradable linker is irradiated is only required to be a wavelength corresponding to each photodegradable linker. For example, in a case of methoxy nitrobenzyl, the degradation efficiency is the highest at 346 nm, and when the degradation efficiency at 346 nm is assumed to be 1, the degradation efficiency is 0.89 at 364 nm, 0.15 at 406 nm, and 0.007 at 487 nm. It is preferable that a wavelength of 300 nm or less be not used because there is a possibility of damaging the sample being sorted. Furthermore, it is preferable that the sample being sorted, particularly, the target biological sample, be irradiated with light at, for example, 30 mW/cm², 100 sec. = 3 J/cm² or the like at which the sample is not damaged. In a case where the target bioparticles are cells, it is said that, depending on the type of the cells, DNA is damaged at an irradiation dose of 500 J/cm² to inhibit the growth of the cells (Callegari, A. J. & Kelly, T. J. Shedding light on the DNA damage checkpoint. Cell Cycle 6, 660-6 (2007)). Furthermore, there is also a report that cytotoxicity does not occur at 42 J/cm² (Masato T, et al, Optical cell separation from three-dimensional environment in photodegradable hydrogels for pure culture techniques, Scientific Reports 4, Article number. 4793 (2014)).

### (vii) Drug Feed Management Unit

The sample sorting device according to the present technology may include the drug feed management unit as necessary. The target biological sample stored in the storage part of the sample sorting system needs to be activated and subjected to gene introduction as necessary, and the drug feed management unit feeds, to the storage part, a drug for activating the target biological sample or a drug for introducing a gene into the target biological sample. Alternatively, the drug feed management unit manages the feed amount of each drug and the like in accordance with a state of the stored target biological sample. As the drugs, known drugs can be used, such as various cytokines (interleukin-2 (IL-2), IL-7, IL-15, IL-21, etc.) and various antibodies (anti-CD3 antibody, anti-CD 28 antibody, etc.) for activation, and various viral vectors (adeno-associated viral vectors, adenoviral vector, retroviral vectors, lentiviral vectors, etc.) into which a plasmid expressing a target gene is introduced for gene introduction, and an appropriate drug can be selected in accordance with the type and state of the target biological sample being sorted. Moreover, a plurality of known drugs can be used in combination.

### (viii) Culture Unit

The sample sorting device according to the present technology may include the culture unit as necessary. It may be necessary to increase the number of target biological samples sorted by the sample sorting system in accordance with how the sample sorting device is used. That is, in the culture unit, the target biological sample stored in the storage part is cultured. Specifically, the temperature in the storage part is controlled to increase the target bioparticles stored in the storage part. Note that the method for adjusting temperature by the culture unit is not particularly limited, and a known method may be employed. For example, with a heating element provided in the storage part, an electric signal for controlling temperature rise/fall may be output from the culture unit to the heating element.

### (ix) Pressure Adjustment Unit

The sample sorting device according to the present technology may include the pressure adjustment unit as necessary. As described above, since the container, the sorting part, and the storage part are airtightly connected to each other in the sample sorting system, a pressure change in the storage part may cause a pressure change in the container and/or the sorting part. The pressure adjustment unit adjusts the pressure in the storage part. Specifically, the pressure adjustment unit is configured to generate negative pressure in the storage part, and examples of the pressure adjustment unit include a piezoelectric element such as a piezo element. Moreover, the pressure adjustment unit is preferably configured to adjust the pressure in the container by adjusting the flow rate of the sample being sorted flowing out from the container.

In the sample sorting device according to the present technology as described above, the container, the sorting part, and the storage part are airtightly connected via a sealing part. Therefore, the target biological sample can be sorted and stored in a sealed space, so that it is possible to improve a purification degree of the sorting of the target biological sample, and it is further possible to prevent the sample sorting system itself from being contaminated by mist containing the target biological sample and/or to prevent other substances from mixing into the sorted target biological sample. This makes the sample sorting device according to the present technology applicable to clinical uses where the target biological sample is required to be high in purity.

The sample sorting device according to the present technology may be a cell sorter airtightly connected with the above-described sample sorting system, and the cell sorter is preferably a closed-system cell sorter, and more preferably a closed-system cell sorter cell sorter used for cell therapy.

The sample sorting system and the sample sorting device according to the present technology may also have the following configurations.
(1) A sample sorting system including: a container in which a sample being sorted is stored; a sorting part that sorts a target biological sample from the sample being sorted; and a storage part used to store the target biological sample in, the container, the sorting part, and the storage part being airtightly connected through a flow channel, and further including a first liquid container that contains a liquid that is caused to flow through the flow channel, in which
   the sorting part includes a first filter module that separates a mixture of solids smaller in size than target bioparticles and a mixture containing the target bioparticles, a second filter module that separates a mixture of solids larger in size than the target bioparticles and the mixture containing the target bioparticles, a first reagent container that contains a reagent used to label the target bioparticles or particles other than the target bioparticles, and a chamber used to temporarily hold a sample, a particle, a reagent, a liquid, or a mixture thereof in the flow channel,
   in the sorting part, a combination of (a), (b), and (c1), a combination of (a), (b), and (c2), or a combination of (a) and (c2) is performed on the sample being sorted flowing from the container, where:
      (a) is removal of the mixture of solids smaller in size than the target bioparticles by the first filter module;
      (b) is removal of the mixture of solids larger in size than the target bioparticles by the second filter module;
      (c1) is processing of labeling the target bioparticles by reaction between the reagent flowing from the first reagent container and the sample being sorted in the chamber and then sorting the labeled particles; and
      (c2) is processing of labeling the particles other than the target bioparticles by reaction between the reagent flowing from the first reagent container and the sample being sorted in the chamber and then removing the labeled particles.
(2) The sample sorting system described in (1), in which
   the labeling is performed using beads, and the reagent used to label particles is a reagent used to bind the beads to the target bioparticles or the particles other than the target bioparticles.
(3) The sample sorting system described in (2), in which
   on the sample being sorted flowing from the container, a combination of (a), (b), and (c1), a combination of (a), (b), and (c2), or a combination of (a) and (c2) is performed, where:
   (a) is removal of the mixture of solids smaller in size than the target bioparticles by the first filter module;
   (b) is removal of the mixture of solids larger in size than the target bioparticles by the second filter module;
   (c1) is processing of binding the beads to the target bioparticles by reaction between the reagent flowing from the first reagent container and the sample being sorted in the chamber and then causing the sample being sorted to flow through the second filter module to sort the target bioparticles bound to the beads as the mixture of solids larger in size than the target bioparticles; and
   (c2) is processing of binding the beads to the particles other than the target bioparticles by reaction between the reagent flowing from the first reagent container and the sample being sorted in the chamber and then causing the sample being sorted to flow through the second filter module to remove the particles bound to the beads as the mixture of solids larger in size than the target bioparticles, and
   in a case where the processing (b) is performed after the processing (c1), processing of removing the beads from the target bioparticles is performed after the processing (c1) and before the processing (b).
(4) The sample sorting system described in (2), in which
   the sorting part includes the first reagent container that contains the reagent used to bind the beads to the target bioparticles, and another reagent container that contains a reagent used to bind the beads to the particles other than the target bioparticles,
   in the sorting part, a combination of (a), (b), (c1), and (c2) or a combination of (a), (c1), and (c2) is performed on the sample being sorted flowing from the container, where:
      (a) is removal of the mixture of solids smaller in size than the target bioparticles by the first filter module;
      (b) is removal of the mixture of solids larger in size than the target bioparticles by the second filter module;
      (c1) is processing of binding the beads to the target bioparticles by reaction between the reagent flowing from the first reagent container and the sample being sorted in the chamber and then causing the sample being sorted to flow through the second filter module to sort the target bioparticles bound to the beads as the mixture of solids larger in size than the target bioparticles; and
      (c2) is processing of binding the beads to the particles other than the target bioparticles by reaction between the reagent flowing from the another reagent container and the sample being sorted in the chamber and then causing the sample being sorted to flow through the second filter module to remove the particles bound to the beads as the mixture of solids larger in size than the target bioparticles, and
      in a case where the processing (b) and/or the processing (c2) is performed after the processing (c1), processing of removing the beads from the target bioparticles is performed after the processing (c1) and before the processing (b) and/or the processing (c2).
(5) The sample sorting system described in any one of (1) to (4), in which
   the first filter module and the second filter module each include a microchannel that separates and guides solids in a sample introduced from an inlet to two different outlets in accordance with sizes of the solids.
(6) The sample sorting system described in any one of (1) to (5), further including a labeling part that labels the sample being sorted with a fluorescent dye, in which
   the labeling part is airtightly connected to at least one of the container or the sorting part.
(7) The sample sorting system described in any one of (1) to (6), in which
   a concentration of the target bioparticles is adjusted.
(8) The sample sorting system described in any one of (1) to (7), in which
   the liquid is introduced from the first liquid container into the container, and the liquid is mixed with the sample being sorted.
(9) A sample sorting device including: a sample sorting system described in any one of (1) to (8); a light irradiation unit that irradiates the sample being sorted with excitation light; a light detection unit that detects fluorescence emitted from the sample being sorted; and an arithmetic processing unit that calculates sorting information on the basis of a detection result from the light detection unit.
(10) The sample sorting device described in (9), including a cell sorter airtightly connected to the sample sorting system.
(11) The sample sorting device described in (10), in which
   the cell sorter includes a closed-system cell sorter.
(12) The sample sorting device described in (10), in which
   the cell sorter includes a closed-system cell sorter used for cell therapy.
(13) The sample sorting device described in any one of (9) to (12), further including a culture unit that adjusts temperature of the storage part.
(14) The sample sorting device described in any one of (9) to (13), further including a drug feed management unit that controls feed of a drug into the storage part.

### REFERENCE SIGNS LIST

- 1: Sample sorting system
- 10: Container
- 20: First filter module
- 21: Second filter module
- 30: First reagent container
- 31: Second reagent container
- 32: Third reagent container
- 40: First liquid container
- 41: Second liquid container
- 50: Chamber
- 60: Storage part
- 70: Disposal part
- 80: Concentration sensor

## Claims

1. A sample sorting system comprising: a container in which a sample being sorted is stored; a sorting part that sorts a target biological sample from the sample being sorted; and a storage part used to store the target biological sample in, the container, the sorting part, and the storage part being airtightly connected through a flow channel, and further comprising a first liquid container that contains a liquid that is caused to flow through the flow channel, wherein
the sorting part includes a first filter module that separates a mixture of solids smaller in size than target bioparticles and a mixture containing the target bioparticles, a second filter module that separates a mixture of solids larger in size than the target bioparticles and the mixture containing the target bioparticles, a first reagent container that contains a reagent used to label the target bioparticles or particles other than the target bioparticles, and a chamber used to temporarily hold a sample, a particle, a reagent, a liquid, or a mixture thereof in the flow channel,
in the sorting part, a combination of (a), (b), and (c1), a combination of (a), (b), and (c2), or a combination of (a) and (c2) is performed on the sample being sorted flowing from the container, where:
(a) is removal of the mixture of solids smaller in size than the target bioparticles by the first filter module;
(b) is removal of the mixture of solids larger in size than the target bioparticles by the second filter module;
(c1) is processing of labeling the target bioparticles by reaction between the reagent flowing from the first reagent container and the sample being sorted in the chamber and then sorting the labeled particles; and
(c2) is processing of labeling the particles other than the target bioparticles by reaction between the reagent flowing from the first reagent container and the sample being sorted in the chamber and then removing the labeled particles.

2. The sample sorting system according to claim 1, wherein
the labeling is performed using beads, and the reagent used to label particles is a reagent used to bind the beads to the target bioparticles or the particles other than the target bioparticles.

3. The sample sorting system according to claim 2, wherein
a combination of (a), (b), and (c1), a combination of (a), (b), and (c2), or a combination of (a) and (c2) is performed on the sample being sorted flowing from the container, where:
(a) is removal of the mixture of solids smaller in size than the target bioparticles by the first filter module;
(b) is removal of the mixture of solids larger in size than the target bioparticles by the second filter module;
(c1) is processing of binding the beads to the target bioparticles by reaction between the reagent flowing from the first reagent container and the sample being sorted in the chamber and then causing the sample being sorted to flow through the second filter module to sort the target bioparticles bound to the beads as the mixture of solids larger in size than the target bioparticles; and
(c2) is processing of binding the beads to the particles other than the target bioparticles by reaction between the reagent flowing from the first reagent container and the sample being sorted in the chamber and then causing the sample being sorted to flow through the second filter module to remove the particles bound to the beads as the mixture of solids larger in size than the target bioparticles, and
in a case where the processing (b) is performed after the processing (c1), processing of removing the beads from the target bioparticles is performed after the processing (c1) and before the processing (b).

4. The sample sorting system according to claim 2, wherein
the sorting part includes the first reagent container that contains the reagent used to bind the beads to the target bioparticles, and another reagent container that contains a reagent used to bind the beads to the particles other than the target bioparticles,
in the sorting part, a combination of (a), (b), (c1), and (c2) or a combination of (a), (c1), and (c2) is performed on the sample being sorted flowing from the container, where:
(a) is removal of the mixture of solids smaller in size than the target bioparticles by the first filter module;
(b) is removal of the mixture of solids larger in size than the target bioparticles by the second filter module;
(c1) is processing of binding the beads to the target bioparticles by reaction between the reagent flowing from the first reagent container and the sample being sorted in the chamber and then causing the sample being sorted to flow through the second filter module to sort the target bioparticles bound to the beads as the mixture of solids larger in size than the target bioparticles; and
(c2) is processing of binding the beads to the particles other than the target bioparticles by reaction between the reagent flowing from the another reagent container and the sample being sorted in the chamber and then causing the sample being sorted to flow through the second filter module to remove the particles bound to the beads as the mixture of solids larger in size than the target bioparticles, and
in a case where the processing (b) and/or the processing (c2) is performed after the processing (c1), processing of removing the beads from the target bioparticles is performed after the processing (c1) and before the processing (b) and/or the processing (c2).

5. The sample sorting system according to claim 1, wherein
the first filter module and the second filter module each include a microchannel that separates and guides solids in a sample introduced from an inlet to two different outlets in accordance with sizes of the solids.

6. The sample sorting system according to claim 1, further comprising a labeling part that labels the sample being sorted with a fluorescent dye, wherein
the labeling part is airtightly connected to at least one of the container or the sorting part.

7. The sample sorting system according to claim 1, wherein
a concentration of the target bioparticles is adjusted.

8. The sample sorting system according to claim 1, wherein
the liquid is introduced from the first liquid container into the container, and the liquid is mixed with the sample being sorted.

9. A sample sorting device comprising: a sample sorting system according to claim 1; a light irradiation unit that irradiates the sample being sorted with excitation light; a light detection unit that detects fluorescence emitted from the sample being sorted; and an arithmetic processing unit that calculates sorting information on a basis of a detection result from the light detection unit.

10. The sample sorting device according to claim 9, comprising a cell sorter airtightly connected to the sample sorting system.

11. The sample sorting device according to claim 10, wherein
the cell sorter includes a closed-system cell sorter.

12. The sample sorting device according to claim 10, wherein
the cell sorter includes a closed-system cell sorter used for cell therapy.

13. The sample sorting device according to claim 9, further comprising a culture unit that adjusts temperature of the storage part.

14. The sample sorting device according to claim 9, further comprising a drug feed management unit that controls feed of a drug into the storage part.
